Europäisches Patentamt

European Patent Office

Office européen des brevets

(11) Publication number: **0 413 637 A1**

## (12) EUROPEAN PATENT APPLICATION

(21) Application number: 90402299.3

(51) Int. Cl.5: **A61K 39/29, C12N 7/08**

(22) Date of filing: **16.08.90**

(30) Priority: **17.08.89 CN 89106580**

(43) Date of publication of application:
**20.02.91 Bulletin 91/08**

(84) Designated Contracting States:
**BE FR SE**

(71) Applicant: **ZHEJIANG ACADEMY OF MEDICAL SCIENCES**
**No 60, Tian Mu Shan Lu**
**Hangzhou(CN)**

(72) Inventor: **Mao,Jiang-sen**
**60 Tian Mu Shan Lu**
**Hangzhou(CN)**
Inventor: **Dong,De-xiang**
**60 Tian Mu Shan Lu**
**Hangzhou(CN)**
Inventor: **Chen,Nian-liang**
**60 Tian Mu Shan Lu**
**Hangzhou(CN)**
Inventor: **Zhang,Hua-yuan**
**60 Tian Mu Shan Lu**
**Hangzhou(CN)**
Inventor: **Huang,Hai-ying**
**60 Tian Mu Shan Lu**
**Hangzhou(CN)**
Inventor: **Zhang,Shu-ya**
**60 Tian Mu Shan Lu**

**Hangzhou(CN)**
Inventor: **Cao,Yi-yun**
**60 Tian Mu Shan Lu**
**Hangzhou(CN)**
Inventor: **Cai,Shao-ai**
**60 Tian Mu Shan Lu**
**Hangzhou(CN)**
Inventor: **Xie,Ru-ying**
**60 Tian Mu Shan Lu**
**Hangzhou(CN)**
Inventor: **Lian,You-hui**
**60 Tian Mu Shan Lu**
**Hangzhou(CN)**
Inventor: **D.Q. Chen**
**60 Tian Mu Shan Lu**
**Hangzhou(CN)**
Inventor: **Zhang,Hang-chun**
**60 Tian Mu Shan Lu**
**Hangzhou(CN)**
Inventor: **Li,Han-min**
**60 Tian Mu Shan Lu**
**Hangzhou(CN)**
Inventor: **Zhu,Ji-ming**
**60 Tian Mu Shan Lu**
**Hangzhou(CN)**

(74) Representative: **Bloch, Gérard et al**
**2, square de l'Avenue du Bois**
**F-75116 Paris(FR)**

(54) **Attenuated live hepatitis A vaccine (H2 Strain) and its method of preparation.**

(57) The strain is obtained by means of isolation of virus from cells of tissue culture. It is characterized by serial passages through tissue cells, culture at low temperature and is adapted to grow in human lung diploid fibroblasts, so as to develop the vaccine ($H_2$strain) for prophylaxis of human hepatitis A.
The monkey model is also established for experimental trials.

EP 0 413 637 A1

## ATTENUATED LIVE HEPATITIS A VACCINE ($H_2$ STRAIN) AND ITS METHOD OF PREPARATION

### Field of Invention

This invention is characterized by a sort of novel biological product containing viral antigen for medical application, especially dealing with an attenuated live hepatitis A vaccine ($H_2$ strain) which is used for prophylaxis of human hepatitis A and its method of preparation.

### Background of Invention

Heptitis A is a global distributed infectious disease which is caused by the pathogen hepatitis A virus (HAV). About 4 billion people in the world are at risk. In China, the infection rate is 90% odd in people over 35 years old. An outbreak of the disease was noted in Shanghai in spring of 1988, and more than 300 thousand contracted HAV. There were above one million hepatitis A patients per year in China. Because of the high incidence and long duration of the disease, it exerts serious effect on human health and economical development.

According to the situation in China, the fundamental procedure for controlling hepatitis A is to develope vaccines. And the key problem is to screen the attenuated viru strains.

In 1986, Provost et al in Merck and Co. Ltd. U.S.A. reported that they obtained an attenuated live hepatitis $F'$ strain, but the effect of immunity in volunteers proved very poor, failing to reveal any pratical usage. The product has neither been subjected to large-scale human test, nor to medium quantity production.

The object of this invention is to provide safe and effective attenuated live hepatitis A vaccine ($H_2$ strain), which is assured to be used for prophylaxis of human hepatitis A, providing a most efficient means for controlling the prevalence of this vital disease.

### Brief Description of the Invention

The strain of attenuated live hepatitis A vaccine ($H_2$ strain) in the invention was derived from the virus isolated from the infected cells through serial passages in tissue culture, being attenuated at low temperature and screened for to get the hepatitis A vaccine, the attenuated $H_2$ strain.

Animal model of susceptible monkey was established for assessment of its effectiveness. Inoculation to both monkey and man evidenced promising safty and immunity. It is the superior hepatitis A attenuated live vaccine in the world at the moment, which exhibits consequence from human inoculation.

### Detailed Description of the Invention

The stool of a patient with hepatitis A was collected and dispersed in PBS to form a 20% suspension. It was extracted through F113-PEG precipitation and gradient centifugation. This extract was then subjected to ultracentrifugation. The sediment containing hepatitis A virus granules of ca 27nm was visualized by immuno electron microscopy. Neonatal monkey kidney cells were used for isolation of virus. The above mentioned preliminarily purified stool extracts was inoculated into monkey kidney monolayer cell culture in 4 separate flasks, rendering absorption for 2 hours at 35-36°C, then incubated in new born calf serum in Eagles and lactoprotein maintenance medium at 35-36°C. The medium was replaced weekly for 6 weeks. The original maintenance medium was discarded. Digestion with trypsin-EDTA was conducted, and cells were collected. Subsequently deep freezing-quick thawing and ultrasonic treatment were performed to extract HAV. The virus in neonatal monkey kidney cells serialy experienced 15 passages, the proliferation of virus for each passage was observed by direct immunofluorescence test. It was further incubated at 32-34°C for 5 successive passages. Thus, the attenuated HAV $H_2$M20 strain was obtained.

This strain was adapted to grow in KMB17 cells which were human lung diploid fibroblasts, and was continuously attenuated through successive passages. The above mentioned $H_2M20$ virus was to KMB17 cells (28th generation), incubated at 32-34° C . One passage was pursued every 28 days for 5 consecutive passages to obtain $K_2M20K5$ strain.

Characteristics of the attenuated Strain

Attenuated H strain was obtained by conducting successive passages in a culture of human lung diploid fibroblasts at about 32° C , and was assessed by a serious of tests in monkey models. In contrast with the master seed virus, the inoculation of the attenuated H strain into monkeys did not result in the elevation of serum glutamic pyruvic transaminase and no pathologic change of hepatic cells was observed. The hepatitis A antigen could not be detected in stool, but specific antibodies would be induced. Experiments of different inoculating routes indicated that this strain would not infect monkey per os. These results demonstrated that the toxity of this strain to monkey was markedly attenuated, but its immunogenicity was rather stable. While maintaining at 36° C for 5 passages, no atavism ever occurred.

When H M20K5 cultured in KMB17 cells for 2 passages, the attenuated live hepatitis A vaccine was developed with material taken from the culture. 139 volunteers without specific antibodies were inoculated with this vaccine, and examined succesively for 6 weeks. No symptoms or signs related to hepatitis A were observed. The level of serium glutamic pyruvic transaminase (SGPT) as well as type 5 isoenzyme of lactate dehydrogenase (LDH5) fell in normal range, indicating adequate safety of this vaccine.

The stool of 4 cases in first batch collected 8-30 days after inoculation was concentrated and examined. Hepatitis A antigen cell cultures in 3 cases. No acute hepatitis case were reported from the surroundings of the inoculators within 12 weeks. It suggested that the quantity of excreted virus after vaccination was very scarce, so that no hepatitis was ever caused by the close contact of the inoculators with other people.

After the first vaccination, the inoculators (specific antibodies was negative originally were subjected to the enzyme linked immunosorbent assay (ELISA), seroconversion (presence of HAV antibodies) occurred 3 weeks later, the positive rate being 95%, and the antibody titer was between 1:1-1:6.4. Positive findings of serum antibodies were recorded in 10 inoculators in follow-up examination 2 years later. The titre of neutralizing antibodies which represents the potential of protection was assayed in 4 subjects and evidence was provided that neutralizing antibodies existed. An effect of 100% protection resulted from vaccination was confirmed by epidemiological survey. The preceding data revealed an excellent immunogenicity of the vaccine.

To date, this M20K5 live attenuated hepatitis A vaccine has been tested among 207 volunteers in 3 groups with satisfactory results.

Up to now, only Dr. Provost (Merck Co. Ltd., U.S.A.) has published the results of trials on a strain, hepatitis A CR326-F'. Of volunteers inoculated, only 6 showed specific antibodies in serum. The commencement of this seroconversion was as late as 7.5 weeks after inculation. No further information about this attenuated strain was provided thereafter. The vaccine produced by the attenuated strain 2 in this invention has proved to hold excellent safety through trials on the largest number of volunteers ever reported under careful observation. Obviously, this live vaccine deriving strain was the most safe one. On the other hand, the rate of seroconversion of antibodies was about 95%, which was far greater than that of F' attenuated strains. Furthermore, with protective effect, it showed special epidemiological significance. These results suggested that the vaccine in this invention which presented its superiority in human vaccination was the best hepatitis vaccine strain.

Examples

Example 1 Isolation of virus.

The stool samples of a hepatitis A patient was collected one week prior to the appearance of icterus. Positive reaction was revealed in hepatitis A IgM test. The patient was healthy before hepatitis A infection, and there was no previous history of chronic or infectious diseases. Fine recovery was acquired after the cure of hepatitis. The stool was suspended into PBS to produce a 20% suspension, then precipitated with F113-PEG and extracted by gradient centrifugation. The extracts were then precipitated by ultrocentrifugation and finally concentrated to 50 folds in PBS. Spherical virus granuels of about 27nm diameter were viewed by immunoelectron microscopy, which could only be coagulated with the convalescent serum of hepatitis A. The neonatal monkey kidney mono-layer cells were used for isolation. A pregnant rhesus monkey was caught from Wu-Yi mountain, and was bred separately. Kidneys were taken from its neonatal offspring immediately, and treated with trypsin to prepare mono-layer cells. They were then digested, frozen and subsequently stored in liquid nitrogen.

The above preliminarily purified stool extracts were inoculated into the monkey kidney mono-layer cell cultures in 4 flasks ($35cm^2$/flask), rendering for 2 hours at $36°C$, treated with Eagles medium containing 2% new born calf serum and maintainance medium containing 0.5% lacto-protein, incubated at $35°C$. The maintenance liquid was replaced weekly for 6 weeks. The maintenance liquid was replaced and discarded, and the culture was further digested with trypsins-EDTA. The cells were collected, and were subjected consecutively to deep freezing-quick thawing and ultrosonic treatment to extract HAV. The virus was passed serially in neonatal monkey kidney monolayer cells and preserved in liquid nitrogen for 15 passages at $35°C$ with above method. The proliferation of virus in each passage was assayed by direct immunofluorescence test. Thereafter, it was further incubated at $32°C$ for 5 passages. The $HAVH_2M20$ ($32°C$) attenuated strain was thus obtained.

The characteristics of the attenuated strain was shown in TableI.

Example 2. Preparation of $H_2M20K5$ strain

This strain was adapted to grow in KMB17 cells, human lung diploid fibroblasts in serial passages for attenuation. M20 virus was inoculated into KMB17 (28th generation) at $32°C$, one passage was performed every 28 days for 5 serial passages. $H_2M20K$ strain was thus obtained.

Example 3 Results of Inoculation of $H_2M20K7$

Attenuated $H_2M20K5$ ($32°C$) thus obtained was further inoculated to KMB17 cells at $32°C$ for 2 serial passages, so as to get experimentally attenuated H2M20K7 ($32°C$) vaccine. The culture fluid of H2M20K7 assayed by direct immunofluorescence test was harvested, experienced twice deep freezing-quick thawing and ultrosonic treatment for 2 minutes, then centrifuged 12000 rpm at $4°C$ for 20 minutes. The supernatant was taken out and bovine serum albumin was added to attain final concentration of 1%. PH was adjusted to 7.2. Rhesus monkey and red face monkey were used in the experiments. Their HAV antibodies were negative. Those monkeys were bred in separate cages 6-9 weeks prior to the experiments. They were randomly divided into 3 groups, i.e. for intraveneous, subcutaneous and per os vaccination, respectively.

Blood specimens were taken from each monkey on the day of inoculation as "0 week specimen". Subcutaneous inoculation was undertaken on the medial side of the thigh. Intraveneous inoculation was performed on the lower limb. The monkeys were bred in seperate cages after inoculation, and intramuscular injection of gentamycin 20,000 unit/time, 2 times daily 3 days was done. The monkeys were fed with conventional forage.

After inoculation, blood specimens 12ml for each time were taken intraveneously every week. One week later, stool samples were collected daily for 50 days. Stool of every 2 days was placed in one container as a single specimen. The whole experiment covered a period of 16 weeks.

The results are shown in table II and table III.

Stool specimen were taken one week prior to the seroconversion or 4 successive specimens were taken after seroconversion for immunoeletronmicroscopic examination. The results were all negative.

The above results showed that the attenuated strain had no infectivity to monkeys inoculated per os, and its toxity to monkeys was significantly lessened.

Example 4. Results of Inoculation on Human Beings

Twelve volunteers of 11-27 years old in the first batch were inoculated with H2M20K7. Two weeks prior to inoculation, hepatitis A antibody response was tested twice. Igm could not be detected. SGPT/ALT, LDH5 and ICD were at normal level, and HBsAg was negative. No abnormality was recorded in physical examination.

Table I

| The characteristics of attenuated H2 strain | |
|---|---|
| 1. shape of granules | spherical |
| j. Size | 2.7nm |
| 3. CsCl$_2$ buoyance density | 1.33g/ml |
| 4. thermotolerance | no obvious influence to infectivity at 60 for 30min, but inactivated at 100 for 5min. |
| 5. titre of infection | 10$^{6.5}$-10$^{7.5}$ TCD 50/ml (KMB17) |
| 6. peak titre time | ca 28days |
| 7. Optimum temperature for proliferation | 33-35° C |
| 8 Site of proliferation: | In the physaliform structure of cells |
| 9. Virus in the extracellular | fluid -fail to secure any passage with the supernatant of extracellular fluid |
| 10. Cytopathology. | no cpF was observed. |

Table II

| Route of inoculation | Monkey number | dosage | antiHAV-antibody | |
|---|---|---|---|---|
| | | | seroconversion | *time of seroconversion (week) |
| I.V. | red face monkey 2 | 0.5x10$^{6.5}$ | 2/2 | 7.7 |
| | rhesus monkey 5 | 0.25x10$^{6.5}$ | 5/5 | 7,8,9,10,11 |
| S.C. | rhesus monkey 5 | 0.25x10$^{6.5}$ | 5/5 | 6,7,8,10 13 |
| oral | red face monkey 3 | 0.5x10$^{6.5}$ | 0/3 | --- |

*By ELISA

Table III

| Serum enzyme in monkeys after inoculation of H2M20K7 (30° C) by different routes | | | | |
|---|---|---|---|---|
| Monkey | Route of inoculation | No. of monkeys | Elevation of SGPT | Elevation of LDH5$_5$ |
| Red face monkey | i.v | 2 | 0/2 | 0/2 |
| rhesus monkey | iv | 5 | 0/5 | 0/5 |
| rhesus monkey | s.c. | 3 | 0/3 | 0/3 |

Table IV

| Assessment of serum enzyme in twelve subiects after inoculation of Hepatitis A vaccine (H2 strain) within 20 weeks. | | | | | |
|---|---|---|---|---|---|
| Method of Assessment | | | | | |
| Subject | manual(1) | Encore(2) | ABA-200(3) | LDH5(%)(4) | ICD(KGA)(5) |
| 1 | 4-14 | ND | 2-15 | 1.9-3.3 | 52-160 |
| 2 | 2-21 | ND | 2-23 | 1.1-4.4 | 75-155 |
| 3 | 5-19 | 14-22 | 14-44 | 1.7-5.3 | 160-220 |
| 4 | 2-21 | 5-11 | 5-22 | 1.2-5.3 | 110-200 |
| 5 | 3-20 | 2-17 | 12-30 | 1.2-5.1 | 132-252 |
| 6 | 7-17 | 8-21 | 14-32 | 1.7-5.9 | 140-285 |
| 7 | 4-17 | 11-20 | 12-29 | 1.7-7.9 | 105-210 |
| 8 | 4-14 | 8-12 | 4-14 | 1.1-3.8 | 115-185 |
| 9 | 4-14 | 3-15 | 5-12 | 1.3-6.4 | 98-190 |
| 10 | 5-20 | 6-16 | 4-19 | 1.4-5.6 | 112-200 |
| 11 | 2-13 | 3-11 | 2-8 | 1.3-3.5 | 142-185 |
| 12 | 1-10 | 2-12 | 2-19 | 2.0-4.8 | 120-150 |
| Note Normal value (1) <30 Iu/l (2) <35Iu/l (3) < 54Iu/l 0.23%-7.36% Normal value ±2SD: 9.58% (5) 238-686KGA | | | | | |

Table IV

| detection of HAV in stool of 4 subjects | | | |
|---|---|---|---|
| Subjects | age | Time of collocting of stoll(wk) | HAAG |
| 1 | 23 | 12,13,14,16,17,19,21,26,28 | negative |
| 2 | 19 | 12,14,16,18,20,21,24,25,26 27,28,29,30 | negative |
| 3 | 27 | 13,14,16,18 | negative |
| 4 | 11 | 8,11,14,21,26-28 | negative |

## Table VI

| Antibody response of 10 subjects after inoculated with $H_2M20K7$ hepatitis A Vaccine | | | | | | | | | | | | | | |
|---|---|---|---|---|---|---|---|---|---|---|---|---|---|---|
| Antibody response | | | | | | | | | | | | | | |
| Name | Prior to inoculation (WK) | After inoculation (WKS) | | | | | | | | | | | | |
| | 2 - 4 | 0 | 1 | 2 | 3 | 4 | 5 | 6 | 8 | 10 | 12 | 16 | 20 | 26 |
| Gu | (1) - | 0 | - | - | + | + | + | + | + | + | + | + | + | + |
| | (2) ND | - | - | - | + | + | + | + | + | + | + | + | ND | ND |
| Zhu | (1) - | - | - | - | + | + | + | + | + | + | + | + | + | + |
| | (2) ND | - | - | - | + | + | + | + | + | + | + | + | ND | ND |
| | | | - | + | + | + | + | + | + | + | + | + | + | |
| Wang | (1) - | - | - | + | + | + | + | + | + | + | + | + | + | |
| | (2) ND | - | - | - | + | + | + | + | + | + | + | ND | ND | |
| Chen | (1) - | - | - | + | + | + | + | + | + | + | + | + | + | |
| | (2) ND | - | - | + | + | + | + | + | + | + | + | ND | ND | |
| Luo | (1) - | - | - | + | + | + | + | + | + | + | + | + | + | |
| | (2) ND | - | - | - | + | + | + | + | - | - | - | ND | ND | |
| Tao | (1) - | - | - | - | + | + | + | + | + | + | + | + | + | |
| | (2) ND | - | - | - | + | + | + | + | + | + | + | ND | ND | |
| Jiang | (1) - | - | - | + | + | + | + | + | + | + | + | + | + | |
| | (2) ND | - | - | + | + | + | + | + | + | + | + | ND | ND | |
| Jiang | (1) - | - | - | - | + | + | + | + | + | + | + | + | + | |
| | (2) ND | - | - | - | + | + | + | + | + | + | + | ND | ND | |
| Yang | (1) - | - | - | + | + | + | + | + | + | + | + | + | + | |
| | (2) ND | - | - | - | + | + | + | + | + | + | + | ND | ND | |
| Xu | (1) - | - | - | + | + | + | + | + | + | + | + | + | + | |
| | (2) ND | - | ND | ND | - | - | + | - | - | - | - | ND | ND | |

Notes: (1) Results of HAAB-E assayed by Zhejiang Academy of Medical Sciences
(2) Results of HAVAB-E (Abbott) assayed by the National Institute for the control of pharmaceutical and Biological Products
ND = Not detected

Table VII

| Antibody response of 12 inoculators | | | | | |
|---|---|---|---|---|---|
| No. of subj. | seroconversion time (WKs) | | Titre of antibodies | | |
| | HAAB (anti) | HAVAB (A) | ELISA | IAHA | |
| | | | 12 wks | 20 wks | 20 wks |
| 1 | 3 | 3 | 4 | 4 | 256 |
| 2 | 3 | 3 | 2 | 4 | 128 |
| 3 | 2 | 3 | 2 | 4 | 128 |
| 4 | 2 | 2 | 4 | 8 | 128 |
| 5 | 2 | 3 | 2 | 2 | 128 |
| 6 | 3 | 3 | 2 | 4 | ND |
| 7 | 2 | 2 | 2 | 2 | 64 |
| 8 | 3 | 3 | 2 | 4 | 128 |
| 9 | 2 | 3 | 2 | 4 | 128 |
| 10 | 2 | 5 | 1 | 2 | 64 |
| 11 | 3 | 3 | 2 | 4 | 64 |
| 12 | 3 | 3 | 4 | 4 | 128 |
| average | 2.5 | 3.0 | 7.2 | 3.6 | 112* |
| Note: | | | | | |
| *GMP | | | | | |

Table VIII

| Response of hepatatis A antibodies in 127 subjects inoculated with vaccine | | |
|---|---|---|
| Before or after inoculation (wk) | HAV antibodies | |
| | No. exam. | No. posit |
| -1 | 127 | 0 |
| 4 | 126 | 126 |
| 6 | 126 | 126 |
| 8 | 126 | 125 |

**Claims**

1. An attenuated live hepatitis A vaccine ($H_2$ strain) and its method of preparation
The strain is obtained by means of isolation of virus from cells of tissue culture. It is characterized by serial passages through tissue cells, culture at low temperature and is adapted to grow in human lung diploid fibroblasts, so as to develop the vaccine ($H_2$ strain) for prophylaxis of human hepatatis A.
The monkey model is also established for experimental trials.

2. According to Claim 1, the attenuated live hepatitis vaccine ($H_2$ strain) and its method of production are characterized in that this strain of live vaccine is produced and attenuated at low temperature 32-34°C and adaptation to grow in human lung diploid cells.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | THE JOURNAL OF INFECTIOUS DISEASES, vol. 159, no. 4, April 1989, pages 621-624; J.S. MAO et al.: "Primary study of attenuated live hepatitis A vaccine (H2 strain) in humans" <br> – – – – – | 1-2 | A 61 K 39/29 <br> C 12 N 7/08 |

|  |  |  | TECHNICAL FIELDS SEARCHED (Int. Cl.5) |
|---|---|---|---|
|  |  |  | A 61 K |

The present search report has been drawn up for all claims

| Place of search | Date of completion of search | Examiner |
|---|---|---|
| The Hague | 29 November 90 | TURMO Y BLANCO C.E. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

&: member of the same patent family, corresponding document